# EUROPÄISCHE PATENTSCHRIFT

(11) **EP 1 377 373 B1**
(45) Veröffentlichungstag und Bekanntmachung des Hinweises auf die Patenterteilung: **04.08.2004**
(21) Anmeldenummer: 02722181.1
(22) Anmeldetag: 06.03.2002
(51) Int. Cl.: B01J 23/89, B01J 27/045, C07D 309/18, C07D 307/28, C07C 31/20

(54) **VERBESSERTES VERFAHREN ZUR HERSTELLUNG VON UNGESÄTTIGTEN CYCLISCHEN ETHERN**
IMPROVED METHOD FOR PRODUCING UNSATURATED CYCLIC ETHERS
PROCEDE AMELIORE DE PREPARATION D'ETHERS CYCLIQUES INSATURES

(30) Priorität: 08.03.2001 DE 10111065
(43) Veröffentlichungstag der Anmeldung: 07.01.2004
(73) Patentinhaber: BASF AKTIENGESELLSCHAFT, 67056 Ludwigshafen (DE)
(72) Erfinder: WEIGL, Hagen, 68526 Ladenburg (DE); EBEL, Klaus, 68623 Lampertheim (DE); HUBER, Sylvia, 64673 Zwingenberg (DE); KUSCHE, Andreas, 67483 Kleinfischlingen (DE); ELLER, Karsten, 67061 Ludwigshafen (DE); HESSE, Michael, 67549 Worms (DE); HÖHN, Arthur, 67281 Kirchheim (DE)
(86) Internationale Anmeldenummer: PCT/EP2002/002425
(87) Internationale Veröffentlichungsnummer: WO 2002/070128

(56) Entgegenhaltungen:
- DE-A- 19 803 368
- US-A- 3 766 179

## Beschreibung

Die vorliegende Erfindung betrifft ein Verfahren zur Herstellung von ungesättigten cyclischen Ethern aus Diolen in flüssiger Phase an mit Schwefel dotierten, cobalt- und edelmetallhaltigen Trägerkatalysatoren unter Zusatz von Wasser.

Aus der DE-A-23 46 943 ist ein Verfahren zur Herstellung ungesättigter cyclischer Verbindungen aus Diolen unter einem Wasserstoffstrom bekannt, in dem als Katalysatoren Gemische aus einem Kupferchromit- oder Kupferträgerkatalysator und einer Wolframoder Heteropolywolframsäure eingesetzt werden. Die Umsätze und Ausbeuten lassen zu wünschen übrig.

Aus der US-A-2 993 910 ist ein Verfahren zur Herstellung von Dihydrofuranen aus 1,4-Butandiolen an Cobaltkatalysatoren, die bei 300 bis 450°C mit Wasserstoff reduziert werden müssen, bekannt.

Aus der DE-A-195 30 993 ist ein Verfahren zur Herstellung von ungesättigten cyclischen Ethern an platindotierten, cobalthaltigen Trägerkatalysatoren bekannt.

Aus der DE-A-198 03 368 ist ein Verfahren zur Herstellung von ungesättigten cyclischen Ethern an mit Schwefel dotierten, cobalt- und edelmetallhaltigen Trägerkatalysatoren bekannt, das zwar befriedigende Standzeiten des Katalysators aufweist, mit dem jedoch nur unbefriedigende Raum-Zeit-Ausbeuten (RZA) erreicht werden.

Aus der US-A-3 766 179 ist ein Gasphasonverfahren zur Herstellung von ungesättigten cyclischen Ethern an kupferhaltigen Trägerkatalysatoren bekannt, bei welchen Wasser und Wasserstoff den Ausgangsmischung zugesetzt wird.

In Anbetracht des Standes der Technik liegt der vorliegenden Erfindung die Aufgabe zugrunde, ein Verfahren zur Herstellung von ungesättigten cyclischen Ethern aus Diolen bereitzustellen, das gute Raum-Zeit-Ausbeuten aufweist.

Die Aufgabe wird erfindungsgemäß gelöst durch ein neues und verbessertes Verfahren zur Herstellung von ungesättigten cyclischen Ethern der allgemeinen Formel I 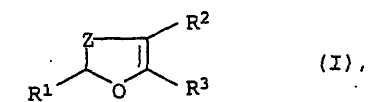 in der
- Z: -(CHR⁴)_{q}- oder -(CHR⁴)_{q}-O-,
- q: 0, 1, 2 oder 3 und
- R¹, R², R³, R⁴: Wasserstoff oder C₁- bis C₄-Alkyl
bedeuten, durch Umsetzung von Diolen der allgemeinen Formel II 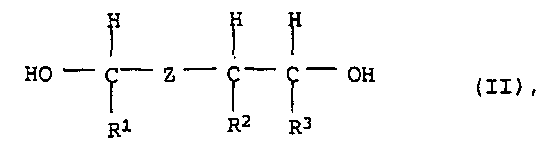 in der Z, R¹, R² und R³ die obengenannten Bedeutungen haben, in flüssiger Phase bei Temperaturen von 150 bis 300°C in Gegenwart eines vor seiner Verwendung nicht durch Reduktion aktivierten, mit Schwefel dotierten, cobalthaltigen Trägerkatalysators, der Cobalt und ein durch Soltränkung aufgebrachtes Edelmetall - aus der Gruppe Platin, Palladium, Rhodium, Iridium, Ruthenium, Osmium, Rhenium oder deren Gemische - auf einem inerten Träger enthält, dadurch gekennzeichnet, dass Wasser zugesetzt wird.

Die Substituenten R¹, R², R³, R⁴, das Zwischenglied Z und der Index q in den Verbindungen I und II haben folgende Bedeutungen:
R¹, R², R³, R⁴ unabhängig voneinander
   - Wasserstoff,
   - C₁- bis C₄-Alkyl wie Methyl, Ethyl, n-Propyl, iso-Propyl, n-Butyl, iso-Butyl, sec.-Butyl und tert.-Butyl, bevorzugt C₁- bis C₃-Alkyl wie Methyl, Ethyl, n-Propyl und iso-Propyl, besonders bevorzugt Methyl und Ethyl,
Z -(CHR⁴)_{q}- oder -(CHR⁴)_{q}-O-,
q 0, 1, 2 oder 3, bevorzugt 0 und 1, besonders bevorzugt 1.

Als Diole II eignen sich z.B. 1,4-Butandiol, 1,5-Pentandiol, 1,6-Hexandiol, Diethylenglykol, bevorzugt 1,5-Pentandiol.

Als ungesättigte cyclische Ether I eignen sich z.B. 3,4-Dihydro-2H-pyrane, 2,3-Dihydrofuran und 1,4-Dioxan, bevorzugt 3,4-Dihydro-2H-pyran.

Bei dem erfindungsgemäßen Verfahren wird zusätzlich zu den geringen Mengen an Reaktionswasser, das bei der Zyklisierung des Diols (III) zum cyclischen Ether (I) entsteht; Wasser zugesetzt. Der Wassergehalt wird in der Regel auf 0,01 bis 25 Gew.-%, bevorzugt auf 0,1 bis 20 Gew.-%, insbesondere bevorzugt auf 0,5 bis 10 Gew.-%, bezogen Diol (II), eingestellt. Dabei kann erfindungsgemäß der Wasserzusatz direkt in die Reaktionsmischung und/oder in den das Diol (II) enthaltenden Zulauf, der auch als Feed bezeichnet wird, erfolgen. Durch den Zusatz von Wasser kann der zusätzliche Vorteil des erfindungsgemäßen Verfahrens ausgenutzt werden, dass ein Diol mit geringerem Wertstoffanteil für das Verfahren verwendet werden kann.

Die Wasserkonzentration im Feed oder im Reaktionsgemisch kann nach der Methode von Karl Fischer nach DIN 51 777 (März 1983) bestimmt werden.

Das erfindungsgemäße Verfahren lässt sich wie folgt durchführen:

Dem Diol (II) wird 0,1 bis 25 Gew.-% Wasser, bezogen auf das Diol(II),zugesetzt. Das Wasser kann jedoch auch in gleicher Menge direkt in das Reaktionsgemisch zugesetzt werden. Man kann diesen das Diol (II) enthaltenden Feed in der Regel mit 0,2 bis 20 Gew.-%, bevorzugt 0,3 bis 10 Gew.-% mit Schwefel dotiertem, cobalthaltigem Trägerkatalysator bei Temperaturen von 150 bis 300°C, bevorzugt 160 bis 240°C umsetzen. Der cobalthaltige Trägerkatalysator kann vorgelegt werden oder während der Reaktion gestuft portioniert in Anteilen von der Gesamtmenge zugegeben werden. Das Reaktionsgemisch sollte während der Umsetzung gleichmäßig vermischt werden, wobei der Energieeintrag so zu wählen ist, dass der Katalysator durch die eingetragene Energie nicht zu sehr belastet wird. Der Wassergehalt des Reaktionsgemischs kann während der Reaktion durch die Bestimmung der Wasserkonzentration in aus dem Reaktionsgemisch gezogenen Proben kontrolliert und gegebenenfalls durch Zugabe von weiterem Wasser in die Reaktionsmischung korrigiert werden. Das entstandene Gemisch aus dem ungesättigten cyclischen Ether (I) und dem Wasser (zugesetztes Wasser und Reaktionswasser) kann diskontinuierlich, bevorzugt kontinuierlich abdestilliert werden. Der bei der Reaktion entstehende ungesättigte cyclische Ether kann gegebenenfalls zur Entfernung des bei der Reaktion entstehenden Wasserstoffs mit unter Reaktionsbedingungen inerten Gasen, wie Stickstoff oder Argon, gestrippt werden. Bei kontinuierlicher Fahrweise kann durch Zufuhr von frischem wasserhaltigem Diol (II)-Feed der Flüssigkeitsstand im Reaktionsgefäß gehalten werden. Der Zusatz von Alkaliund/oder Erdalkalimetallverbindungen, um den Gehalt an destillativ nur schwer vom ungesättigten 3,4-Dihydro-2H-pyran abtrennbaren gesättigten cyclischen Ether zu senken, ist bei dem erfindungsgemäßen Verfahren nicht erforderlich.

Als cobalt- und edelmetallhaltige, mit Schwefel dotierte Trägerkatalysatoren eignen sich die Oxide des Cobalts oder metallisches Cobalt und ein oder mehrere Elemente der Edelmetalle aus der Gruppe Platin, Palladium, Rhodium, Iridium, Ruthenium, Osmium, Rhenium oder deren Gemische, bevorzugt Platin, Palladium, Rhenium oder deren Gemische, besonders bevorzugt Platin, Palladium oder deren Gemische sowie gegebenenfalls 0,001 bis 10 Gew.-%, bevorzugt 0,1 bis 5 Gew.-%, besonders bevorzugt 0,5 bis 3 Gew.-% basische Alkali-, Erdalkalimetallsalze, Scandium, Vanadin, Chrom, Mangan, Eisen, Nickel, Kupfer, Zink, Germanium, Zinn, Blei, Antimon, Wismut oder deren Gemische (Verbindung A), bevorzugt Lithium, Kalium, Natrium, Calcium, Strontium, Barium, Mangan, Eisen, Nickel, Kupfer, Zink, Zinn, Antimon oder deren Gemische, besonders bevorzugt Kalium, Natrium, Mangan, Eisen, Nickel, Kupfer, Zink oder deren Gemische auf einem porösen Träger.

Der Gewichtsanteil des Cobalts im Trägerkatalysator beträgt in der Regel 1 bis 70 Gew.-%, bevorzugt 5 bis 50 Gew.-%, besonders bevorzugt 10 bis 40 Gew.-%.

Der Gewichtsanteil der Elemente aus der Gruppe Platin, Palladium Rhodium, Iridium, Ruthenium, Osmium oder Rhenium, bevorzugt Platin, Palladium oder Rhenium, beträgt 0,001 bis 2 Gew.-%, bevorzugt 0,05 und 1 Gew.-%, besonders bevorzugt 0,01 bis 0,5 Gew.-%, bezogen auf den Trägerkatalysator.

Der Gewichtsanteil des Schwefels (berechnet als S) liegt zwischen 0,015 und 2 Gew.-%, bevorzugt 0,1 bis 1 Gew.-%, insbesondere bevorzugt 0,3 bis 0,6 Gew.-%, bezogen auf den Trägerkatalysator

Zur Bestimmung des Schwefelgehalts der Katalysatoren wird der Trägerkatalysator in Salzsäure gelöst und mit hypophosphoriger Säure behandelt. Der dabei gebildete Schwefelwasserstoff wird im N₂-Strom ausgetrieben, in ammoniakalischer Cadmiumacetatlösung aufgefangen und jodometrisch bestimmt.

Die Trägerkatalysatoren weisen in der Regel ein Gewichtsverhältnis von Cobalt zum Edelmetall von 10 : 1 bis 10.000 : 1 und von Edelmetall zum Schwefel von 100 : 1 bis 1 : 100 auf.

Als Träger eignen sich inerte Träger wie SiO₂, Al₂O₃, TiO₂, ZrO₂, Zeolithe aller Art wie kleinporige Zeolithe, z.B. α-Zeolith, mittelporige Zeolithe, z.B. ZSM-5, ZSM-11, Ferrierit, großporige Zeolithe, z.B. Faujasite, β-Zeolithe, Mordenit, Offretit, hydrothermal hergestellte Phosphate wie AlPO und SAPO, Aktivkohlen oder Erdalkalioxide, bevorzugt SiO₂, ZrO₂ und Zeolithe, besonders bevorzugt SiO₂, wobei in der Regel ein Gewichtsverhältnis von Cobalt zum SiO₂ im Trägerkatalysator 1 : 20 bis 1 : 1 vorliegt.

Die Trägerkatalysatoren weisen in der Regel eine BET-Oberfläche von 1 bis 600 m²/g, bevorzugt 10 bis 500 m²/g, besonders bevorzugt 50 bis 400 m²/g auf.

Die Porosität der Trägerkatalysatoren liegt in der Regel bei 0,01 bis 1,5 ml/g, bevorzugt 0,1 bis 1,2 ml/g, besonders bevorzugt 0,2 bis 1 ml/g.

Die erfindungsgemäß verwendeten Trägerkatalysatoren werden hergestellt, indem man zunächst Cobalt, dann das Edelmetall in Form eines Sols auf den Träger aufbringt und anschließend mit Schwefel dotiert.

Die Herstellung der cobalthaltigen Trägerkatalysatoren ist allgemein bekannt. Vorteilhaft ist die Tränkung des porösen Trägermateriales mit einer löslichen Cobaltverbindung (z.B. eines Nitrits, Nitrats, Sulfits, Sulfats, Carbonats, Hydroxids, von Carboxylaten, Halogeniden, Halogeniten, Halogenaten u.a.), gegebenenfalls gleichzeitig oder nacheinander mit einer ebenfalls löslichen Verbindung A (z.B. als Nitrit, Nitrat, Sulfit, Sulfat, Carbonat, Hydroxid, Carboxylate, Halogenide, Halogenite, Halogenate u.a.), und anschließender thermischer Zersetzung des Anions zum Oxid. Eine weitere Möglichkeit ist das Mischen einer Cobalt-Verbindung mit dem Trägermaterial (trocken oder in Suspension, insbesondere durch Sprühtrocknung), gegebenenfalls gleichzeitig mit einer chemischen Verbindung A, Verdichtung des Materials (z.B. durch Verkneten, gegebenenfalls Zugabe eines geeigneten Verformungshilfsmittels), Formgebung durch Extrudieren, Trocknung und anschließender Calcinierung bei Temperaturen von 200 bis 1300°C, bevorzugt 300 bis 1000°C, besonders bevorzugt 400 bis 800°C.

Das Edelmetall - aus der Gruppe Platin, Palladium, Rhodium, Iridium, Ruthenium, Osmium, Rhenium oder deren Gemische - wird sodann durch das Besprühen des noch heißen Trägers oder durch Tränkung des Trägers mit einem zuvor hergestellten Sol auf den Träger aufgebracht, wobei zuvor gegebenenfalls durch die vorangegangene Tränkung agglomerierte Katalysatormasse zerkleinert wird.

Das Edelmetallsol ist ein kolloidaler Stoff und kann nach bekannten Methoden, z.B. ausgehend von Metallsalzen, in denen das Edelmetall in einer Oxidationsstufe größer Null vorliegt, hergestellt werden. Es können z.B. wäßrige Lösungen der Chloride, Acetate oder Nitrate des Metalls verwendet werden. Es sind aber auch andere Edelmetallsalze verwendbar; eine Beschränkung hinsichtlich des Anions besteht nicht. Als Reduktionsmittel lassen sich organische Verbindungen wie Ethanol, Methanol, Carbonsäuren und deren Alkalisalze sowie anorganische Verbindungen wie N₂H₄ oder NaBH₄ verwenden. Bevorzugt sind Hydrazin N₂H₄ und Ethanol. Die Teilchengröße der Metallpartikel im Sol hängt hierbei von der Stärke des eingesetzten Reduktionsmittels und vom verwendeten Metallsalz ab. Die Sole lassen sich durch Zugabe von organischen Polymeren wie Polyaminen, Polyvinylpyrrolidon oder Polyacrylaten stabilisieren, wobei Polyvinylpyrrolidon PVP bevorzugt ist, Die Solherstellung kann aber auch nach anderen in der Literatur beschriebenen Vorgehensweisen durchgeführt werden. Bönnemann et al. (Angew. Chemie, 103 (1991), 1344) beschreiben beispielsweise die Herstellung von stabilen Metallsolen durch Reduktion von Metallsalzen mit (C₈H₁₇)₄N[BEtH₃].

Die Aufbringung der Sole auf den Träger kann nach verschiedenen Techniken erfolgen, die wie die prinzipielle Herstellung der Katalysatoren und die Katalysatoren selbst ausführlich in der DE 198 03 368, auf die hier ausdrücklich Bezug genommen wird, beschrieben sind.

Es ist vorteilhaft, dass die aus DE 198 03 368 bekannten Katalysatoren vor ihrer Anwendung im erfindungsgemäßen Verfahren nicht durch die Behandlung mit Wasserstoff oder anderen Reduktionsmitteln, wie Hydrazin, aktiviert werden müssen.

Die ungesättigten cyclischen Ether I sind wertvolle Schutzgruppen für Alkohole.

### Beispiele

### Herstellung des Katalysators A

3400 ml einer Lösung aus 3,25 kg Co(NO₃)₂ · 6 H₂O in Wasser wurde mit 2,5 kg SiO₂-Pulver (Wasseraufnahme 1,5 ml/g) ca. 2 h verrührt, 16 h bei 120°C getrocknet und 2 h bei 500°C calciniert.

Diese Masse wurde sodann mit 2,9 1 eines Edelmetall-Sols, hergestellt durch Mischen von 14,2 g Platinnitrat in 4,5 1 destilliertem Wasser mit 32 g Polyvinylpyrrolidon und 1,93 1 Ethanol und 4 h Erhitzen unter Rückfluß, getränkt, anschließend bei 100°C im Vakuum getrocknet und 2 h bei 500°C unter N₂-Atomosphäre calciniert. Der so hergestellte Katalysator enthält 0,12 Gew.-% PtO₂. 4,6 g dieser cobalt- und platinhaltigen Masse wurde sodann mit einer Lösung von (NH₄)₂S in Wasser getränkt, anschließend bei 100°C im Vakuum getrocknet und 2 h bei 350°C calciniert.

Weitere Einzelheiten zur Herstellung und zu den Eigenschaften des Katalysatos 1 sind Tabelle 2 zu entnehmen.

**Tabelle 1**

| Katalysator | Menge Edelmetall-Sol [ml] | Edelmetallgehalt [Gew.-%] | (NH₄)₂S [g] | Schwefelgehalt [Gew.-%] |
|---|---|---|---|---|
| A | 2900 | 0,10 | 0,04 | 0,34 |

### Beispiele 1 bis 4

1,5 1 1,5-Pentandiol, das nach Wasserzusatz einen Wassergehalt gemäß Tabelle 1 aufwies, und 45 g des Katalysators A wurden vorgelegt. Unter Rühren wurde bis zur unteren Grenze des Temperaturintervalls erwärmt, wobei die Reaktion unter Wasserstoffentwicklung anspringt. Das entstehende 3,4-Dihydro-2H-pyran/Wasser-Gemisch wurde kontinuierlich abdestilliert und die Temperatur im Sumpf wurde während der Reaktion so nachgeregelt, dass die pro Stunde anfallende Destillatmenge konstant blieb (40 - 50 ml). Gleichzeitig wurde innerhalb der aus Tabelle 2 ersichtlichen Zeiten durch wasserhaltiges 1,5-Pentandiol mit einem Wassergehalt gemäß Tabelle 2 kontinuierlich zudosiert, um den Stand im Reaktionskolben konstant zu halten. Nach Phasentrennung des Destillats erhielt man 3,4-Dihydro-2H-pyran. Die jeweils bestimmte Raum-Zeit-Ausbeute ist in Tabelle 1 angegeben.

### Vergleichsbeispiel V 5

1,5 1 1,5-Pentandiol und 45 g des Katalysators A wurden vorgelegt und unter Rühren bis zur unteren Grenze des Temperaturintervalls erwärmt, wobei die Reaktion unter Wasserstoffentwicklung anspringt. Die Reaktionsdurchführung unterschied sich von den erfindungsgemäßen Beispielen 1 bis 4 dadurch, dass kein Wasser zugesetzt wurde. Die Versuchsdurchführung entsprach der in der DE 198 03 368 beschriebenen Versuchsdurchführung. Nach Phasentrennung des Destillats erhielt man 3,4-Dihydro-2H-pyran. Die bestimmte Raum-Zeit-Ausbeute ist in Tabelle 1 angegeben.

**Tabelle 2**

| Bsp. | Katalysator | Temperatur [°C] | Dauer [h] | Menge zugesetzten. Wassers [g] | Wassergehalt [Gew.-%], bezogen auf 1,5-Pentandiol, | RZA [kg/l·h] |
|---|---|---|---|---|---|---|
| 1 | A | 170-230 | 29 | 27,6 | 2 | 0,1 |
| 2 | A | 170-230 | 51 | 54,3 | 3 | 0,12 |
| 3 | A | 170-230 | 52 | 115,5 | 5 | 0,09 |
| 4 | A | 170-230 | 83 | 268,2 | 10 | 0,07 |
| V5 | A | 170-230 | 780 | - | * | 0,021 |
| RZA = Raum-Zeit-Ausbeute * = kein Wasserzusatz, nur Reaktionswasser | | | | | | |

Die in Tabelle 1 zusammengestellten Versuchsergebnisse zeigen deutlich, dass der erfindungsgemäße Zusatz von Wasser Beispiele 1 bis 4 zu deutlich besseren Raum-Zeit-Ausbeuten führt.

## Patentansprüche

1. Verfahren zur Herstellung von ungesättigten cyclischen Ethern der allgemeinen Formel I 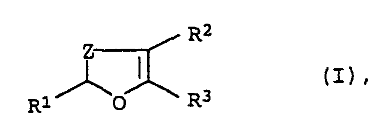 in der
Z -(CHR⁴)_{q}- oder -(CHR⁴)_{q}-O-,
q 0, 1, 2 oder 3 und
R¹, R², R³, R⁴ Wasserstoff oder C₁- bis C₄-Alkyl
bedeuten, durch Umsetzung von Diolen der allgemeinen Formel II 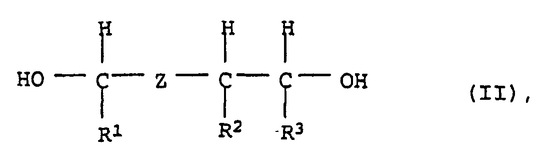 in der Z, R¹, R² und R³ die obengenannten Bedeutungen haben, in flüssiger Phase bei Temperaturen von 150 bis 300°C in Gegenwart eines vor seiner Verwendung nicht durch Reduktion aktivierten, mit Schwefel dotierten, cobalthaltigen Trägerkatalysators, der Cobalt und ein durch Soltränkung aufgebrachtes Edelmetall - aus der Gruppe Platin, Palladium, Rhodium, Iridium, Ruthenium, Osmium, Rhenium oder deren Gemische - auf einem inerten Träger enthält, **dadurch gekennzeichnet, dass** Wasser zugesetzt wird.

2. Verfahren zur Herstellung von ungesättigten cyclischen Ethern nach Anspruch 1, **dadurch gekennzeichnet, dass** dem Feed und/oder während der Reaktion dem Reaktionsgemischs Wasser zugesetzt wird.

3. Verfahren zur Herstellung von ungesättigten cyclischen Ethern nach Anspruch 1 oder 2, **dadurch gekennzeichnet, dass** durch den Wasserzusatz ein Wassergehalt von 0,01 bis 25 Gew.-%, bezogen auf das Diol (II), eingestellt wird.

4. Verfahren zur Herstellung von ungesättigten cyclischen Ethern I nach den Ansprüchen 1 bis 3, **dadurch gekennzeichnet, dass** der Wassergehalt 0,1 bis 20 Gew.-%, bezogen auf das Diol (II),beträgt.

5. Verfahren zur Herstellung von ungesättigten cyclischen Ethern I nach den Ansprüchen 1 bis 4, **dadurch gekennzeichnet, dass** der Wassergehalt 0,5 bis 10 Gew.-%, bezogen auf das Diol (II),beträgt.

6. Verfahren zur Herstellung von ungesättigten cyclischen Ethern I nach den Ansprüchen 1 bis 5, **dadurch gekennzeichnet, dass** die cobalthaltigen Trägerkatalysatoren 1 bis 70 Gew.-% Cobalt, 0,001 bis 2 Gew.-% eines oder mehrerer Edelmetalle und 0,015 bis 2 Gew.-% Schwefel enthalten.

7. Verfahren zur Herstellung von ungesättigten cyclischen Ethern nach den Ansprüchen 1 bis 6, **dadurch gekennzeichnet, daß** 1,5-Pentandiol zu 3,4-Dihydro-2H-pyran umgesetzt wird.

## Claims

1. A process for preparing unsaturated cyclic ethers of the formula I 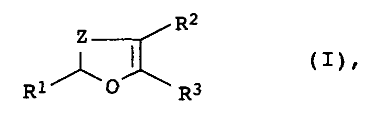 where
Z is -(CHR⁴)_{q}- or -(CHR⁴)_{q}-O-,
q is 0, 1, 2 or 3 and
R¹,R²,R³,R⁴ are hydrogen or C₁-C₄-alkyl,
by reacting diols of the formula II 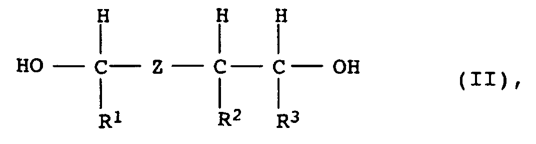 where Z, R¹, R² and R³ are as defined above, in the liquid phase at from 150 to 300°C in the presence of a cobalt-containing supported catalyst which has not been activated by reduction before use, is doped with sulfur and comprises cobalt and a noble metal selected from the group consisting of platinum, palladium, rhodium, iridium, ruthenium, osmium, rhenium or a mixture thereof applied by sol impregnation on an inert support, wherein water is added.

2. A process for preparing unsaturated cyclic ethers as claimed in claim 1, wherein water is added to the feed and/or to the reaction mixture during the reaction.

3. A process for preparing unsaturated cyclic ethers as claimed in claim 1 or 2, wherein a water content of from 0.01 to 25% by weight, based on the diol (II), is set by the addition of water.

4. A process for preparing unsaturated cyclic ethers I as claimed in any of claims 1 to 3, wherein the water content is from 0.1 to 20% by weight, based on the diol (II).

5. A process for preparing unsaturated cyclic ethers I as claimed in any of claims 1 to 4, wherein the water content is from 0.5 to 10% by weight, based on the diol (II).

6. A process for preparing unsaturated cyclic ethers I as claimed in any of claims 1 to 5, wherein the cobalt-containing supported catalyst comprises from 1 to 70% by weight of cobalt, from 0.001 to 2% by weight of one or more noble metals and from 0.015 to 2% by weight of sulfur.

7. A process for preparing unsaturated cyclic ethers as claimed in any of claims 1 to 6, wherein 1,5-pentanediol is converted into 3,4-dihydro-2H-pyran.

## Revendications

1. Procédé de préparation d'éthers cycliques insaturés de formule générale I 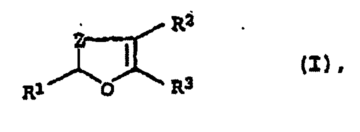 dans laquelle
Z représente -(CHR⁴)_{q}- ou -(CHR⁴)_{q}-O-,
q représente 0, 1, 2 ou 3 et
R¹, R², R³, R⁴ représentent un atome d'hydrogène ou un groupe alkyle en C₁ à C₄
par conversion en phase liquide de diols de formule générale II 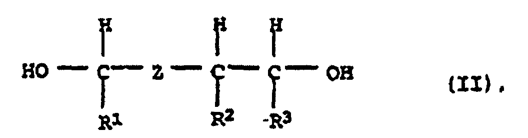 dans laquelle Z, R¹, R² et R³ ont les significations citées ci-dessus, à des températures de 150 à 300°C en présence d'un catalyseur porteur contenant du cobalt dopé au soufre, non activé par réduction avant son utilisation, qui contient du cobalt et un métal noble - choisi parmi le groupe platine, palladium, rhodium, iridium, ruthénium, osmium, rhénium ou leurs composés - amenés par trempage en solution sur un porteur inerte, **caractérisé en ce que** de l'eau est ajoutée.

2. Procédé de préparation d'éthers cycliques insaturés selon la revendication 1, **caractérisé en ce que** de l'eau est ajoutée au produit d'alimentation et/ou, pendant la réaction, au mélange réactionnel.

3. Procédé de préparation d'éthers cycliques insaturés selon la revendication 1 ou 2, **caractérisé en ce qu'**une teneur en eau comprise entre 0,01 et 25 % en poids par rapport au diol (II) est mise en oeuvre par l'adjonction d'eau.

4. Procédé de préparation d'éthers cycliques I insaturés selon les revendications 1 à 3, **caractérisé en ce que** la teneur en eau par rapport au diol (II) est comprise entre 0,1 et 20 % en poids.

5. Procédé de préparation d'éthers cycliques I insaturés selon les revendications 1 à 4, **caractérisé en ce que** la teneur en eau par rapport au diol (II) est comprise entre 0,5 et 10 % en poids.

6. Procédé de préparation d'éthers cycliques I insaturés selon les revendications 1 à 5, **caractérisé en ce que** les catalyseurs porteurs contenant du cobalt contiennent 1 à 70 % en poids de cobalt, 0,001 à 2 % en poids d'un ou de plusieurs métaux nobles et 0,015 à 2 % en poids de soufre.

7. Procédé de préparation d'éthers cycliques insaturés selon les revendications 1 à 6, **caractérisé en ce que** du 1,5-pentanediol est converti en 3,4-dihydro-2H-pyranne.
